# FASCICULE DE BREVET EUROPEEN

(11) **EP 3 234 802 B1**
(45) Date de publication et mention de la délivrance du brevet: **24.05.2023**
(21) Numéro de dépôt: 15830810.6
(22) Date de dépôt: 17.12.2015
(51) Int. Cl.: G06F 17/18, A61B 5/00, G06Q 50/22, G06Q 10/04, G06Q 10/06, G06Q 50/06, H03M 7/30, G16H 40/67, H04L 12/28, H04L 12/64, H04L 69/04

(54) **PROCÉDÉ DE TRANSMISSION DE DONNÉES ISSUES D'UN CAPTEUR**
VERFAHREN ZUR ÜBERTRAGUNG VON DATEN VON EINEM SENSOR
METHOD FOR TRANSMITTING DATA FROM A SENSOR

(30) Priorité: 19.12.2014 FR 1462866
(43) Date de publication de la demande: 25.10.2017
(73) Titulaire: Orange, 92130 Issy-les-Moulineaux (FR)
(72) Inventeur: CIBAUD, David, 38120 Saint Egreve (FR); BOUZID, Marie-Jeanne, 38330 Montbonnot Saint Martin (FR); BERENGUER, Marc, 38420 Revel (FR)
(86) Numéro de dépôt international: PCT/FR2015/053598
(87) Numéro de publication internationale: WO 2016/097633

(56) Documents cités:
- US-A1- 2009 204 008
- RAJASEKARAN M PALLIKONDA ET AL: "Elderly patient monitoring system using a wireless sensor network", TELEMEDICINE AND E-HEALTH,, vol. 15, no. 1, 2009, pages 73-79, XP002579874, ISSN: 1530-5627, DOI: 10.1089/TMJ.2008.0056
- JIE YIN ET AL: "Sensor-Based Abnormal Human-Activity Detection", IEEE TRANSACTIONS ON KNOWLEDGE AND DATA ENGINEERING, IEEE SERVICE CENTER, LOS ALAMITOS, CA, US, vol. 20, no. 8, août 2008 (2008-08), pages 1082-1090, XP011224113, ISSN: 1041-4347, DOI: 10.1109/TKDE.2007.1042
- NOURY ET AL: "MONITORING BEHAVIOR HOME USING A SMART FALL SENSOR AND POSITION SENSORS", 1ST ANNUAL INTERNATIONAL IEEE-EMBS SPECIAL TOPIC CONFERENCE ON MICROTECHNOLOGIES IN MEDICINE AND BIOLOGY. PROCEEDINGS (CAT. NO.00EX451) IEEE PISCATAWAY, NJ, USA, PISCATAWAY, NJ : IEEE OPERATIONS CENTER, 2000, US, 12 octobre 2000 (2000-10-12), pages 607-610, XP007908797, DOI: 10.1109/MMB.2000.893857 ISBN: 978-0-7803-6603-9
- ALEMDAR H ET AL: "Wireless sensor networks for healthcare: A survey", COMPUTER NETWORKS, ELSEVIER SCIENCE PUBLISHERS B.V., AMSTERDAM, NL, vol. 54, no. 15, 28 octobre 2010 (2010-10-28), pages 2688-2710, XP027289934, ISSN: 1389-1286 [extrait le 2010-05-11]

## Description

### DOMAINE TECHNIQUE

L'invention appartient au domaine de la domotique et concerne particulièrement la collecte et la supervision de données issues de capteurs d'un environnement.

### ART ANTÉRIEUR

De nombreux systèmes, tels que des systèmes domotiques, utilisent des capteurs répartis dans un environnement. Ces capteurs sont utilisés par de tels systèmes pour mesurer différentes valeurs, comme par exemple une température ou l'activité d'une personne par exemple. Des capteurs peuvent aussi être utilisés pour détecter une présence, l'ouverture ou la fermeture d'une porte ou encore l'état d'un dispositif motorisé. Certains types de capteurs peuvent également être portés par un utilisateur ou un animal domestique. Il s'agit par exemple de bracelets ou de montres connectés et adaptés pour être reconnus par le système domotique et transmettre des informations sur les déplacements de son porteur.

Les données capturées par de tels capteurs sont généralement transmises à un serveur capable par exemple de commander des actions en réponse à certains évènements détectés, de lever des alertes ou de mettre en forme certaines données pour une présentation à un utilisateur.

La transmission des données entre le capteur et le serveur est généralement réalisées par l'intermédiaire d'un réseau sans fil comme par exemple un réseau sans fil de type 3G, Wifi ou Bluetooth.

Certains capteurs effectuent des mesures en continu et rendent compte de l'évolution de leur environnement. C'est le cas par exemple pour des capteurs tels que des capteurs de température, de lumière, de vitesse du vent ou encore d'accélération. Ce fonctionnement en continu génère un flot de données continu qui nécessite parfois une bande passante importante. En particulier, certains capteurs d'accélération embarqués dans des objets tels que des smartphones, des bracelets ou des montres connectées mesurent les accélérations selon 3 axes à des fréquences élevées, générant ainsi un flot de données important qui nécessite une bande passante importante provoquant régulièrement des congestions sur le réseau. Lorsque les données capturées ne peuvent être traitées par le capteur lui-même et doivent être transmises à un autre équipement, des congestions peuvent apparaître sur le réseau.

De façon à éviter un épuisement trop rapide des ressources énergétiques du capteur, celui-ci est souvent doté d'une mémoire dans laquelle sont enregistrées les données capturées, le contenu de la mémoire étant transmis régulièrement vers le serveur. Ainsi, l'interface réseau n'est sollicitée que par intermittence et le capteur peut limiter la consommation d'énergie liée à la transmission des données. Toutefois, cette approche nécessite d'équiper le capteur avec une quantité de mémoire adaptée à la fréquence d'acquisition des données, ce qui n'est pas toujours économiquement viable. Le document "Elderly patient monitoring system using a wireless sensor network", RAJASEKARAN M PALLIKONDA ET AL. TELEMEDICINE AND E-HEALTH,, vol. 15, no. 1, 2009, pages 73-79, XP002579874,ISSN: 1530-5627, DOI: 10.1089/TMJ.2008.0056 divulgue un système de l'art antérieur où des capteurs de paramètres physiologiques utilisent des seuils afin de n'envoyer à un serveur que des valeurs de capteur potentiellement associées à un danger pour un patient.

Ainsi, il existe bien un besoin pour une solution moins consommatrice en capacité réseau ou en capacité mémoire pour la collecte et la supervision par un serveur, de données transmises en continu par un capteur.

### RÉSUMÉ DE L'INVENTION

À cet effet, l'invention propose un procédé de transmission vers un dispositif de supervision tel que défini dans la revendication 1.

Lorsque le capteur obtient une nouvelle donnée, par exemple lorsqu'il obtient une mesure de température au moyen d'une sonde thermique à laquelle il est associé, la valeur de cette nouvelle donnée est comparée à une valeur prédite par un modèle de prédiction. Le modèle de prédiction est déterminé à partir de données mesurées sur une période précédente. Il peut s'agir par exemple d'une loi affine, d'une régression linéaire ou encore par exemple une régression polynomiale. Un indicateur d'écart est calculé à partir de la mesure et de la valeur de cette mesure prédite par le modèle de façon à valider ou infirmer l'adéquation de la valeur mesurée avec le modèle. L'indicateur d'écart peut être par exemple une différence entre la valeur mesurée et la valeur prédite, ou encore une valeur issue d'une étude statistique prenant en compte plusieurs mesures, comme par exemple un test de χ² (Khi-2) qui permet de valider l'adéquation d'une série de données avec un modèle. Lorsque la valeur mesurée est en adéquation avec la valeur prédite par le modèle, elle n'est pas transmise. De cette façon, seules les données qui ne correspondent pas aux prédictions sont transmises. Le procédé permet ainsi de réduire considérablement le nombre de mesures à transmettre lorsque les valeurs mesurées sont en adéquation avec un modèle de prédiction.

Par exemple, lorsqu'un modèle de prédiction modélise l'évolution de températures sur une période de 24 heures, un capteur mettant en oeuvre le procédé peut confronter des relevés de températures avec ce modèle et transmettre uniquement les relevés qui ne sont pas en adéquation avec le modèle. Le capteur limite ainsi l'utilisation du réseau et augmente son autonomie. Les valeurs en adéquation avec le modèle étant ignorées, le procédé ne requière pas d'espace de stockage important.

Selon un mode particulier de réalisation, le procédé est tel qu'il comporte au préalable les étapes suivantes :
- Transmission vers le dispositif de supervision des données acquises par le au moins un capteur sur une période temporelle prédéterminée, et
- Réception, en provenance du dispositif de supervision, d'un modèle de prédiction représentatif de données transmises.

Les données mesurées sur une période sont transmises à un dispositif de supervision, comme par exemple un serveur disposant de capacités de traitement. En retour, un dispositif mettant en oeuvre le procédé reçoit un modèle de prédiction représentatif des données transmises. Considérons par exemple un capteur connecté mettant en oeuvre le procédé de transmission. Le capteur mesure des températures sur une période de 24 heures et transmet vers un serveur les relevés correspondant pour la période. En retour, le capteur reçoit un modèle de prédiction représentatif des données mesurées sur la période. De cette façon, le capteur dispose d'un modèle de prédiction basé sur une observation des mesures sur une période précédente lui permettant de sélectionner efficacement les données à transmettre. La détermination du modèle de prédiction étant réalisée par un autre équipement, le capteur ne nécessite pas de capacités de traitement importantes.

L'invention concerne également un procédé de supervision tel que défini dans la revendication 3.

Le serveur de supervision peut ainsi analyser, mémoriser et/ou mettre à disposition d'autres équipements toutes les valeurs acquises par un ou plusieurs capteurs, y compris les données qui n'ont pas été reçues sur une période d'observation. Pour cela, le serveur utilise un modèle de prédiction représentatif de données reçues précédemment et permettant d'obtenir une valeur prédite pour les mesures qui n'ont pas été reçues. De cette façon, les données qui sont en adéquation avec les valeurs correspondantes prédites par le modèle n'ont pas à être transmises au serveur de supervision car elles peuvent être obtenues par le serveur à partir du modèle. Le procédé permet ainsi de limiter de façon importante le nombre de données échangées entre un capteur et un serveur de supervision.

Selon un mode particulier de réalisation, le procédé de supervision est tel qu'il comporte en outre les étapes suivantes :
- Réception de données en provenance du au moins un capteur sur une période temporelle prédéterminée,
- Calcul d'un modèle prédictif représentatif des données reçues sur la période, et
- Transmission du modèle prédictif au au moins un capteur.

Un dispositif de supervision qui met en oeuvre un tel procédé de supervision reçoit des données en provenance d'un appareil de mesure, d'un détecteur ou par exemple d'un capteur tel qu'un capteur de mouvement, de température, de vent ou encore de lumière, les données reçues correspondant à une période temporelle déterminée. Il peut s'agir d'une période d'une heure, d'une journée d'une semaine ou d'un mois par exemple. À la fin de la période considérée, le dispositif de supervision détermine un modèle prédictif représentatif de l'évolution des mesures sur la période. Par exemple, le dispositif de supervision peut modéliser les données par une loi affine, une régression linéaire ou polynomiale, une loi de normale ou tout autre outil statistique adapté pour modéliser une série de données. Le dispositif de supervision transmet ensuite les caractéristiques du modèle au capteur. Le modèle peut être représentatif de données issues de plusieurs capteurs.

De cette façon, le capteur n'a pas besoin de disposer d'une capacité de traitement importante car la détermination du modèle est prise en charge par le dispositif de supervision. D'autre part, le dispositif peut conserver une copie du modèle afin de reconstituer un jeu de mesures complet lors de périodes ultérieures à partir d'une part de données transmises par le capteur qui ne sont pas en adéquation avec le modèle et d'autre part de la copie du modèle qu'il conserve.

Le procédé de supervision permet de prendre en compte des données reçues pour mettre à jour le modèle de prédiction. Ainsi, le modèle de prédiction peut être adapté lorsque les relevés transmis par les capteurs évoluent. De cette façon, un équipement peut interroger le modèle de prédiction du serveur pour connaître la valeur des différentes mesures effectuées par le capteur, qu'elles aient ou non été transmises par ce dernier. D'autre part, le modèle étant représentatif des données mesurées sur la période, il n'est pas nécessaire de conserver les données ayant servie à son élaboration. Ainsi, seul le modèle de prédiction est conservé et l'espace de stockage nécessaire à la mémorisation des données est très limité.

Le modèle est mis à jour à partir de données qui ne sont pas en adéquation avec le modèle dont dispose le capteur, sinon elles n'auraient pas été transmises. Lorsque le modèle est mis à jour, un serveur mettant en oeuvre le procédé de supervision peut transmettre ce nouveau modèle au capteur correspondant de façon à réduire le nombre de mesures à transmettre pour des périodes ultérieures.

Selon une réalisation particulière, le procédé est tel que l'étape de mise à jour est réalisée lorsque la fréquence de réception de nouvelles données est supérieure à un seuil.

La fréquence des données reçues augmente lorsque les données mesurées par un capteur mettant en oeuvre le procédé de transmission ne sont plus en adéquation avec le modèle. Le procédé permet ainsi de mettre à jour le modèle et de le transmettre au capteur lorsque qu'il n'est plus en adéquation avec les données mesurée.

Une version mise à jour du modèle de prédiction peut également être transmise au capteur lorsque des données sont reçues à intervalle régulier d'une façon récurrente d'une période sur l'autre. Par exemple, si alors qu'une période de mesure de 24h est considérée et que des données sont reçues de façon quotidienne toujours à une certaine heure, le procédé peut détecter une inadéquation du modèle et transmettre une nouvelle version mise à jour au capteur.

L'invention concerne également un dispositif de transmission tel que défini dans la revendication 6.

Selon une réalisation particulière, le dispositif de transmission est tel que le module de communication est en outre adapté pour recevoir, un modèle de prédiction représentatif des données transmises en provenance du dispositif de supervision.

Selon un autre aspect, l'invention se rapporte à un dispositif de supervision tel que défini dans la revendication 8.

Selon une réalisation particulière, le dispositif de supervision est tel qu'il comporte en outre un calculateur adapté pour calculer un modèle prédictif représentatif de données reçues sur une période prédéterminée, et que le module de communication est en outre adapté pour transmettre le modèle prédictif à au moins un dispositif de transmission.

L'invention se rapporte aussi à un terminal comprenant un dispositif de transmission tel que décrit ci-dessus.

L'invention se rapporte aussi à un serveur comprenant un dispositif de supervision tel que décrit ci-dessus.

L'invention concerne également un programme d'ordinateur comportant les instructions pour l'exécution du procédé de transmission et/ou du procédé de supervision, lorsque le programme est exécuté par un processeur.

L'invention concerne aussi un support d'informations lisible par un processeur sur lequel est enregistré un programme d'ordinateur comprenant des instructions pour l'exécution des étapes du procédé de transmission et/ou du procédé de supervision.

Les différents modes ou caractéristiques de réalisation précités peuvent être ajoutés indépendamment ou en combinaison les uns avec les autres, aux étapes du procédé de transmission ou du procédé de supervision.

Les serveurs, terminaux, dispositifs, programmes et supports d'information présentent au moins des avantages analogues à ceux conférés par les procédés correspondants décrit ci-dessus.

### LISTE DES FIGURES

D'autres caractéristiques et avantages de l'invention apparaîtront plus clairement à la lecture de la description suivante d'un mode de réalisation particulier, donné à titre de simple exemple illustratif et non limitatif, et des dessins annexés, parmi lesquels :
- La figure 1 illustre de manière simplifiée une architecture adaptée pour la mise en oeuvre des procédés de transmission et de supervision selon un mode de réalisation particulier de l'invention.
- La figure 2 illustre les étapes principales du procédé de transmission selon un mode particulier de réalisation de l'invention.
- Les figures 3a et 3b illustrent les principales étapes du procédé de supervision selon une réalisation particulière de l'invention.
- Les figures 4a et 4b représentent deux séries de mesures acquises par un capteur sur une période temporelle sur lesquelles sont superposées deux courbes illustrant un modèle prédictif pour lesdites périodes.
- La figure 5 illustre une architecture simplifiée d'un dispositif de transmission selon un mode particulier de réalisation de l'invention.
- La figure 6 illustre une architecture simplifiée d'un dispositif de supervision selon un mode particulier de réalisation de l'invention.
- La figure 7 est un diagramme illustrant la mise en oeuvre du procédé de transmission sur 3 périodes consécutives.

### DESCRIPTION DÉTAILLÉE

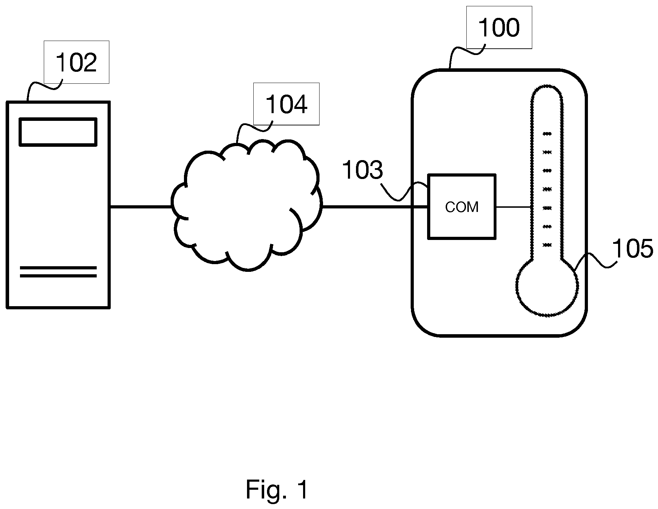

La **figure 1** illustre de manière simplifiée l'architecture d'un système de supervision adapté pour la mise en oeuvre des procédés de transmission et de supervision selon un mode de réalisation particulier de l'invention. Elle représente en particulier un dispositif de transmission **100** comprenant un capteur thermique **105** et une interface réseau **103** adaptée pour transmettre par exemple des relevés de température mesurées par le capteur **105** à un serveur **102** par l'intermédiaire d'un réseau **104.** Le dispositif de transmission peut dans un autre exemple de réalisation comporter plusieurs capteurs de mesure de différents types. Ce dispositif de transmission peut par exemple être un terminal de communication de type « smartphone » dans lequel sont intégré des capteurs. Il peut aussi être un simple dispositif de transmission de température comme illustré sur la **figure 1****.** L'interface réseau **103** est également apte à recevoir des données transmises par le serveur **102,** comme par exemple des modèles prédictifs calculés par le serveur **102** à partir de données transmises par le capteur 105. Le serveur **102** peut être hébergé par exemple sur une passerelle domestique d'un réseau local et communiquer avec le dispositif de transmission **100** par l'intermédiaire d'un réseau local. Différentes technologies réseau peuvent être utilisées, comme par exemple un réseau Wifi, Ethernet ou encore Bluetooth. Il peut également être hébergé au sein d'un
réseau de communication de type Internet et communiquer avec le dispositif de transmission **100** par l'intermédiaire d'un réseau Internet ou 3G par exemple. Bien que l'invention soit décrite ici sur un exemple de capteur de température, elle est applicable à différents types de capteurs et trouve une application particulièrement avantageuse avec des capteurs avec une fréquence de mesure élevée, comme par exemple des accéléromètres ou des gyroscopes. Le système de supervision représenté ici ne comporte qu'un seul dispositif de transmission comprenant un seul capteur **105** mais selon d'autres exemples, le dispositif de transmission peut comporter une pluralité de capteurs et le système de supervision peut comporter une pluralité de dispositifs de transmission **100.**

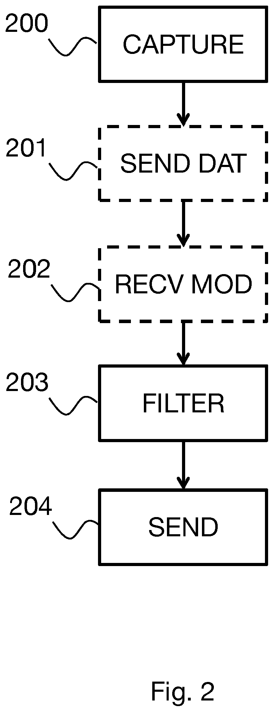

La **figure 2** illustre les étapes principales du procédé de transmission selon un mode particulier de réalisation de l'invention.

Lors d'une étape **200,** le dispositif de transmission **100** obtient des données mesurées par le capteur de température **105.** Dans d'autres modes de réalisation, les données peuvent provenir par exemple et de façon non exhaustive, de capteurs adaptés pour mesurer des accélérations, des vitesses angulaires ou encore des champs magnétiques. Les données peuvent également être issues de plusieurs capteurs ou de plusieurs instances d'un même type de capteur. Par exemple, les données peuvent provenir d'un accéléromètre adapté pour mesurer des accélérations selon 3 axes.

Lors d'une étape **203,** les données obtenues du capteur thermique **105** sont confrontées à un modèle prédictif stocké par exemple dans une mémoire du dispositif. Le modèle peut aussi être stocké dans une base de données du réseau 104 et consultable par le dispositif ou les capteurs.

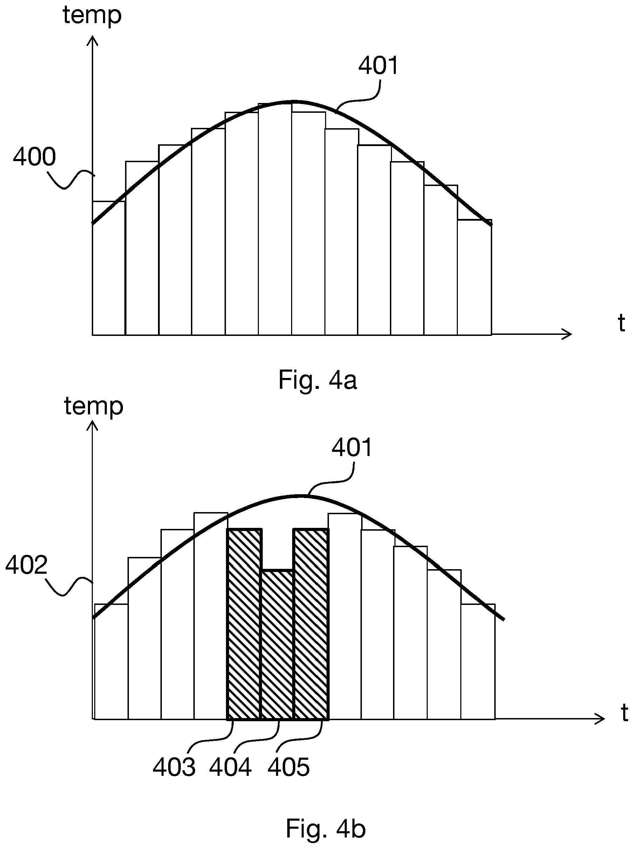

Le modèle prédictif utilisé est représentatif de l'évolution des données mesurées par le capteur. Ce modèle peut être par exemple une fonction numérique de type affine qui, pour un instant donné, permet de prédire la valeur d'une mesure. Selon d'autres modes de réalisation, les données sont modélisées par exemple par une régression linéaire ou polynomiale ou toute autre fonction mathématique ou statistique adaptée pour décrire l'évolution de la série de données mesurées. La **figure 4a** représente par exemple un diagramme en bâtons illustrant 12 relevés de températures **400** sur une période de 24 heures, au rythme d'une mesure toutes les deux heures. Dans cet exemple, les données sont modélisées par une loi normale dont la moyenne et la variance sont déterminées, par exemple, par essais successifs. Ce modèle représentatif des données est représenté sur la figure par la courbe **401.**

Un indicateur d'écart est calculé à partir d'une donnée obtenue du capteur **105** et de sa valeur prédite selon le modèle prédictif de façon à valider ou infirmer l'adéquation de la valeur mesurée avec la valeur prédite par le modèle. L'adéquation peut être vérifiée par exemple en mesurant l'écart entre la valeur mesurée et la valeur prédite, ou selon un mode particulier de réalisation, à partir d'une valeur issue d'une étude statistique prenant en compte plusieurs mesures, ou encore par exemple à partir d'un test de χ² (Khi-2) permettant de valider l'adéquation d'une série de données avec un modèle.

À l'étape **204,** les données qui ne sont pas en adéquation avec le modèle sont transmises au serveur **102** par l'intermédiaire du réseau. Les données qui sont en adéquation avec le modèle sont quant à elles ignorées, de façon à réduire la quantité de données transmises sur le réseau.

La **figure 4b** représente un diagramme en bâtons illustrant 12 relevés de températures **402** sur une période de 24 heures, au rythme d'une mesure toutes les deux heures. Dans cet exemple, les données sont modélisées par la même loi normale que celle représentée sur la **figure 4a****,** représentée par la courbe **401.** Dans cet exemple, les relevés **403, 404** et **405** ne correspondent plus au modèle prédictif et la comparaison de l'indicateur d'écart avec un seuil de tolérance prédéterminé désigne ces relevés comme devant être transmis au serveur. Ainsi, seuls les relevés **403, 404** et **405** sont transmis sur la période.

Selon un mode de réalisation particulier, le procédé de transmission comprend une phase d'initialisation pendant laquelle aucun modèle prédictif n'est disponible pour le dispositif de transmission **100.** Durant l'étape **201,** toutes les mesures obtenues pendant une période d'initialisation sont transmises au serveur **102** car, faute de modèle prédictif disponible, il n'est pas possible de calculer un indicateur d'écart. À l'issue de cette première période, le serveur 102 transmet un modèle prédictif calculé à partir des données transmises par le dispositif de transmission **100** pendant la période d'initialisation. Ainsi, un modèle prédictif représentatif des données mesurées sur la période d'initialisation est reçu à l'étape **202.** Ce modèle peut alors être utilisé pour vérifier l'adéquation des données issues du capteur **100** lors des périodes ultérieures.

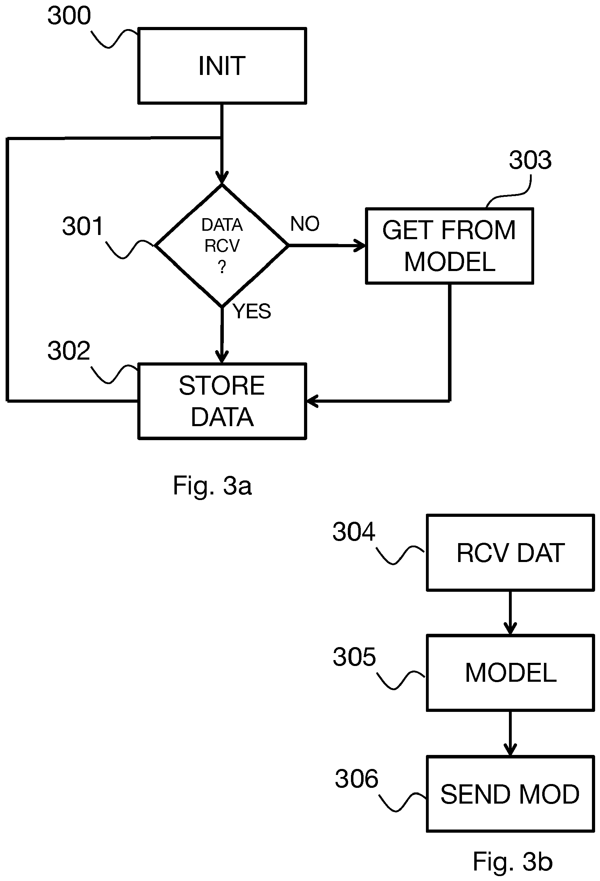

La **figure 3a** représente des étapes pouvant être réalisées pour mettre en oeuvre le procédé de supervision selon un mode particulier de réalisation de l'invention. Le procédé est par exemple mis en oeuvre sur le serveur **102** décrit en référence à la **figure 1****.**

À l'étape **300** le serveur **102** initialise une tâche de supervision des données issues du dispositif de transmission **100** visant par exemple à mémoriser dans une base de données les températures relevées par le capteur de température **105** au cours de la journée.

Pour chaque plage horaire, le serveur vérifie à l'étape **301** si une donnée en provenance du dispositif de transmission a été reçue. Pour cela, le serveur mémorise par exemple dans une mémoire vive les données reçues et la plage horaire à laquelle elles correspondent. Si une donnée est trouvée dans la mémoire pour une plage horaire, cette donnée est mémorisée dans la base de données à l'étape **302** et la plage horaire suivante peut être traitée.

Lorsqu'à l'étape **301** une donnée n'est pas trouvée dans la mémoire vive pour une plage horaire donnée, le serveur **102** évalue à l'étape **303** un modèle prédictif représentatif de données précédemment mesuré par le capteur. L'évaluation de ce modèle permet au serveur **102** d'obtenir une valeur prédite pour la plage horaire considérée lorsqu'une donnée n'est pas reçue.

La **figure 3b** illustre des étapes du procédé de supervision selon une réalisation particulière de l'invention.

Lors d'une étape initiale **304,** le serveur **102** reçoit des mesures relatives à une période donnée en provenance du dispositif de transmission **100.** Ces données sont par exemple transmises par le dispositif de transmission **100** lors de l'étape d'initialisation **201** décrite en référence à la **figure 2** et correspondent aux relevés effectués par le capteur sur une première période d'initialisation. Selon une réalisation particulière, ces données sont stockées par le serveur dans une base de données ou une mémoire vive.

À partir de ces données, le serveur **102** calcule à l'étape **305** un modèle prédictif représentatif des données reçues sur la période considérée. Pour cela, le serveur peut déterminer des paramètres d'une fonction numérique, comme par exemple des paramètres d'une fonction basée sur une loi affine, normale ou encore une fonction polynomiale. Le nombre et la valeur des paramètres sont choisis de manière à obtenir une fonction dont les valeurs s'approchent des mesures transmises par le dispositif de transmission. Le choix des paramètres peut être réalisé selon différentes techniques d'optimisation connues de l'homme du métier, comme par exemple une méthode d'optimisation aux moindres carrés ou une technique de type splines.

Selon un autre mode de réalisation particulier, les données à modéliser sont segmentées en une pluralité de plages temporelles, chacune des plages étant modélisée indépendamment par une fonction numérique et des paramètres, lesdits paramètres étant déterminés par exemple par une méthode d'optimisation de type moindre carrés ou splines.

À l'étape **306,** le modèle est transmis au dispositif de transmission **100,** par exemple sous la forme d'une fonction numérique et de paramètres calculés à l'étape précédente.

De cette façon, le procédé de supervision décharge le dispositif de transmission de l'étape de calcul du modèle qui est particulièrement coûteuse en temps de calcul.

À l'issue de l'étape **306,** une copie du modèle prédictif est conservée sur le serveur **102** de sorte que par la suite, selon les étapes décrites en référence à la **figure 3a****,** si un équipement interroge le serveur pour consulter une mesure transmise par le capteur, ou pour les besoins d'une tâche de supervision, le serveur puisse évaluer le modèle prédictif pour connaître la valeur d'une mesure qui n'a pas été reçue. Ainsi, selon une réalisation particulière, il n'est pas nécessaire de conserver les données à partir desquelles a été calculé le modèle et les données reçues à l'étape **304** peuvent être supprimées afin de ne pas occuper inutilement d'espace de stockage sur le serveur.

Selon une réalisation particulière, le jeu de données initialement utilisé pour calculer le modèle est mémorisé dans une base de données. À la réception d'une nouvelle donnée en provenance du dispositif de transmission, la donnée correspondante est remplacée par la nouvelle donnée dans la base de données. Le serveur utilise ce jeu de données modifié pour calculer un nouveau modèle prédictif. Pour cela, le serveur exécute à nouveau l'étape **305** à partir du jeu de données modifié mémorisé dans la base de données.

Selon un mode particulier de réalisation, lorsque le modèle est mis à jour par le serveur **102** suite à la réception d'une nouvelle donnée, ce modèle mis à jour est transmis au dispositif de transmission **100.** Le procédé permet ainsi d'améliorer l'adéquation des données issues du capteur **105** avec le modèle prédictif de façon à réduire davantage la quantité des données échangées entre le dispositif de transmission **100** et le serveur **102.**

Selon un mode particulier de réalisation, le serveur **102** mesure la fréquence à laquelle des mesures sont transmises par le dispositif de transmission **100.** Pour cela, le serveur calcule par exemple un indicateur prenant en compte la fréquence de réception des mesures sur une période et le nombre de mesures transmises initialement par le dispositif de transmission **100** lors de l'étape **304.** Les mesures n'étant transmises que lorsqu'elles ne sont pas en adéquation avec le modèle, plus le serveur reçoit de mesures, moins le modèle est en adéquation avec les données mesurées. Ainsi, lorsque l'indicateur est supérieur à un seuil prédéterminé, le serveur recalcule un modèle prédictif sur la base des dernières données reçues pour la période et transmet ce nouveau modèle au dispositif de transmission.

Selon une réalisation particulière, le serveur **102** analyse la répartition temporelle des données reçues. Lorsque par exemple des données reçues sont regroupées sur un intervalle temporel restreint par rapport à la période d'observation, seule la partie du modèle correspondant à cet intervalle temporel est mise à jour à partir des nouvelles données et transmise au dispositif de transmission. Par exemple lorsque que le dispositif de transmission de température **100** utilise un modèle prédictif de 24 heures pour filtrer l'envoi des relevés de températures et que le serveur **102** reçoit des relevés correspondant à l'intervalle temporel [12 heure - 14 heure], le serveur peut en déduire que le modèle utilisé par le capteur n'est plus adapté pour cette tranche horaire. Le serveur **102** calcule alors un nouveau modèle prédictif représentatif des données reçues sur l'intervalle [12 heure - 14 heure] et transmet ce nouveau modèle au dispositif de transmission **100.** Le procédé permet ainsi de mettre à jour le modèle prédictif sans qu'il soit nécessaire de le recalculer entièrement. Ce mode de réalisation préserve ainsi les ressources de calcul du serveur et la bande passante pour transmettre le modèle.

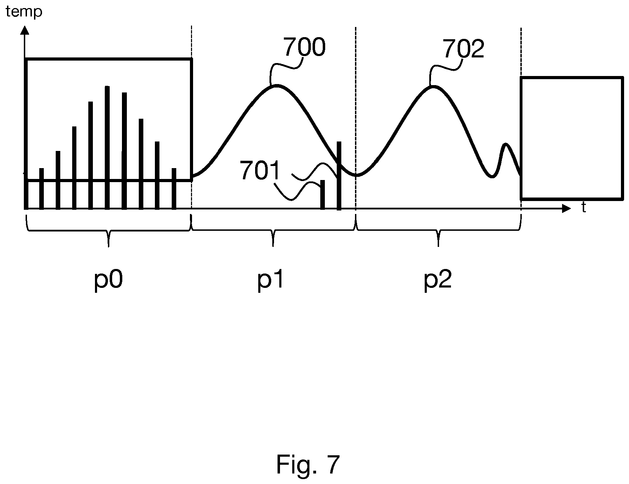

La **figure 7** illustre de manière synthétique sous la forme d'un diagramme les relevés de températures transférées entre le dispositif de transmission **100** et le serveur **102.** La figure montre trois périodes de fonctionnement **P0, P1** et **P2,** chaque période ayant par exemple une durée de 24h. La période **P0** est une période d'initialisation. Durant cette période, le dispositif de transmission **100** ne dispose pas d'un modèle prédictif et toutes les mesures sont transmises au serveur **102.** À l'issue de la période **P0,** le serveur **102** calcule et transmet au dispositif de transmission un modèle prédictif selon les étapes **301** et **302** décrites précédemment. Lors de la période **P1**, le dispositif de transmission met en oeuvre un filtrage des mesures à partir du modèle prédictif **700** en vérifiant l'adéquation des mesures avec le modèle selon l'étape **203.** Seules les mesures qui ne sont pas en adéquation avec le modèle, comme par exemple les mesures **701,** sont transmises au serveur. À l'issue de la période **P1,** le serveur analyse la répartition des mesures reçues et décide de mettre à jour le modèle prédictif à partir de ces nouvelles mesures et de le transmettre au dispositif de transmission **100** de manière à réduire le trafic. Lors de la période **P2,** le modèle mis à jour **702** est utilisé par le dispositif de transmission **100** pour filtrer les mesures. Le modèle étant en adéquation avec les données mesurées, aucune mesure n'est transférée.

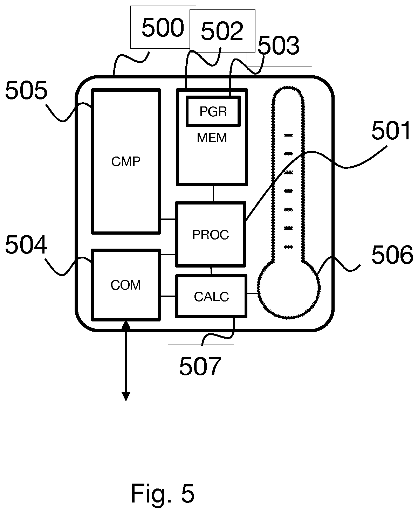

La **figure 5** illustre un dispositif **500** mettant en oeuvre le procédé de transmission selon un mode particulier de réalisation de l'invention. Le dispositif comprend un espace de stockage **502,** par exemple une mémoire MEM, une unité de traitement **501** équipée par exemple d'un processeur PROC. L'unité de traitement peut être pilotée par un programme **503,** par exemple un programme d'ordinateur PGR, mettant en oeuvre le procédé de transmission tel que décrit dans l'invention en référence à la **figure 2****,** et notamment les étapes de calcul d'un indicateur d'écart entre la valeur de la nouvelle donnée et une valeur prédite pour cette donnée par un modèle de prédiction représentatif de données précédemment acquises, et de transmission de la nouvelle donnée vers le dispositif de supervision lorsque l'indicateur d'écart est supérieur à un seuil. Selon une réalisation particulière, le dispositif met également en oeuvre les étapes de transmission vers le dispositif de supervision des données acquises par le au moins un capteur sur une période temporelle prédéterminée, et de réception, en provenance du dispositif de supervision, d'un modèle de prédiction représentatif des données transmises.

À l'initialisation, les instructions du programme d'ordinateur **503** sont par exemple chargées dans une mémoire RAM (Random Access Memory en anglais) avant d'être exécutées par le processeur de l'unité de traitement **501.** Le processeur de l'unité de traitement **501** met en oeuvre les étapes du procédé de transmission selon les instructions du programme d'ordinateur **503.**

Pour cela, le dispositif comprend, outre la mémoire **502,** des moyens de communication **504** (COM) permettant au dispositif de se connecter à un réseau de télécommunication et d'échanger des données avec d'autres dispositifs par l'intermédiaire du réseau de télécommunications, et en particulier de transmettre des mesures vers un serveur et de recevoir un modèle prédictif. Selon une réalisation particulière, le dispositif comprend en outre un module d'acquisition d'une mesure **506** adapté pour capturer par exemple une grandeur physique liée à l'environnement ou à des mouvements. Par exemple, le module d'acquisition **506** est un capteur de température, un accéléromètre, un gyroscope, un compas, un anémomètre ou encore un module d'interfaçage adapté pour connecter un capteur distant. Cette unité d'interfaçage peut correspondre par exemple à une interface USB (Universal Serial Bus), Bluetooth, Ethernet ou encore par exemple à un bus de communication. Le dispositif comprend également un calculateur **507** (CALC) adapté pour calculer un indicateur d'écart entre la valeur de la nouvelle donnée et une valeur prédite pour cette donnée par un modèle de prédiction représentatif des données précédemment acquises par le module d'acquisition **506,** un comparateur **505** (CMP) adapté pour comparer l'indicateur d'écart à un seuil de tolérance et permettant au module de communication **504** de transmettre une nouvelle donnée vers le dispositif de supervision lorsque l'indicateur d'écart est supérieur au seuil.

Selon un mode particulier de réalisation, le dispositif peut être intégré dans un terminal ou une passerelle domestique.

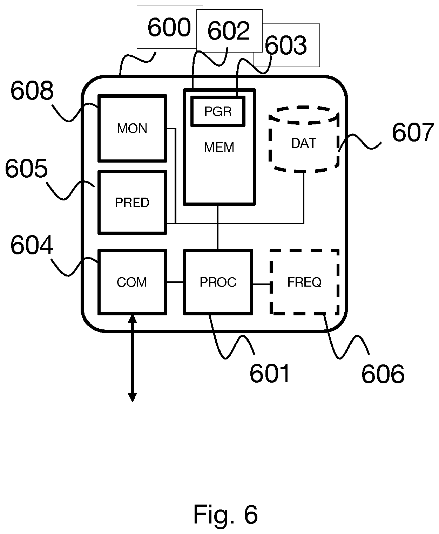

La **figure 6** illustre un dispositif **600** mettant en oeuvre le procédé de supervision selon un mode particulier de réalisation de l'invention. Le dispositif comprend un espace de stockage **602,** par exemple une mémoire MEM, une unité de traitement **601** équipée par exemple d'un processeur PROC. L'unité de traitement peut être pilotée par un programme **603,** par exemple un programme d'ordinateur PGR, mettant en oeuvre le procédé de supervision tel que décrit dans l'invention en référence à la **figure 3****,** et notamment les étapes de réception de données en provenance d'au moins un capteur; de prise en compte, pour remplacer une valeur de capteur non reçue, d'une valeur prédite par un modèle de prédiction représentatif de données précédemment reçues tant qu'une nouvelle donnée, dont un indicateur d'écart entre sa valeur et la valeur prédite pour cette donnée est supérieur à un seuil, n'est pas reçue ; de calcul d'un modèle prédictif représentatif des données reçues sur une période prédéterminée; et de transmission du modèle prédictif vers au moins un capteur. Selon une réalisation particulière, le dispositif met également en oeuvre les étapes de mise à jour d'un modèle prédictif à partir de nouvelles données reçues.

À l'initialisation, les instructions du programme d'ordinateur **603** sont par exemple chargées dans une mémoire RAM (Random Access Memory en anglais) avant d'être exécutées par le processeur de l'unité de traitement **601.** Le processeur de l'unité de traitement **601** met en oeuvre les étapes du procédé de transmission selon les instructions du programme d'ordinateur **603.**

Pour cela, le dispositif comprend, outre la mémoire **602,** des moyens de communication **604** (COM) permettant au dispositif de se connecter à un réseau de télécommunication et d'échanger des données avec d'autres dispositifs par l'intermédiaire du réseau de télécommunications, et en particulier de recevoir des données de mesures en provenance d'un dispositif de transmission et de transmettre un modèle prédictif représentatif de données reçues. Le dispositif comprend également un calculateur **605** (PRED) adapté pour calculer un modèle prédictif représentatif des données reçues sur une période prédéterminée et un module de supervision **608** (MON) adapté pour prendre en compte, pour remplacer une valeur de capteur non reçue, une valeur prédite tant qu'une nouvelle donnée dont un indicateur d'écart entre sa valeur et la valeur prédite pour cette donnée est supérieur à un seuil, n'est pas reçue par le module de communication.

Selon une réalisation particulière, le dispositif comporte un module d'analyse **606** de la fréquence de réception de données par le module de communication et une base de données **607** adaptée pour mémoriser un ensemble de données de mesures reçues sur une période d'observation.

Selon un mode particulier de réalisation, le dispositif peut être intégré dans un serveur ou une passerelle domestique.

## Revendications

1. Procédé de transmission vers un dispositif de supervision, de données collectées par au moins un capteur comportant les étapes suivantes à l'acquisition d'une nouvelle donnée par le au moins un capteur :
- Calcul (203) d'un indicateur d'écart entre la valeur de la nouvelle donnée et une valeur prédite pour cette donnée par un modèle de prédiction représentatif de données précédemment acquises, et
- Transmission (204) de la nouvelle donnée vers un dispositif de supervision lorsque l'indicateur d'écart est supérieur à un seuil, le procédé étant **caractérisé par** l'étape supplémentaire de
- Réception d'un modèle de prédiction mis à jour à la base de la nouvelle donnée, un calcul ultérieur d'un indicateur d'écart se basant sur le modèle de prédiction mis à jour.

2. Procédé selon la revendication 1 **caractérisé en ce qu'**il comporte au préalable les étapes suivantes :
- Transmission (201) vers le dispositif de supervision de données acquises par le au moins un capteur sur une période temporelle prédéterminée, et
- Réception (202), en provenance du dispositif de supervision, d'un modèle de prédiction représentatif des données transmises.

3. Procédé de supervision par un serveur de supervision à partir de données issues d'au moins un capteur, **caractérisé en ce qu'**il prend en compte, pour remplacer une valeur de capteur non reçue, une valeur prédite par un modèle de prédiction représentatif de données précédemment reçues tant qu'une nouvelle donnée, dont un indicateur d'écart entre sa valeur et la valeur prédite pour cette donnée est supérieur à un seuil, n'est pas reçue et **en ce qu'**il comporte, en outre, à la réception d'une nouvelle donnée issue du au moins un capteur, une étape de mise à jour du modèle prédictif et une étape de transmission du modèle mis à jour vers le au moins un capteur.

4. Procédé de supervision selon la revendication 3, **caractérisé en ce qu'**il comporte les étapes suivantes :
- Réception (300) de données en provenance du au moins un capteur sur une période temporelle prédéterminée,
- Calcul (301) d'un modèle prédictif représentatif des données reçues sur la période, et
- Transmission (302) du modèle prédictif vers le au moins un capteur.

5. Procédé selon la revendication 3, **caractérisé en ce que** l'étape de mise à jour du modèle est réalisée lorsque la fréquence de réception de nouvelles données est supérieure à un seuil.

6. Dispositif de transmission vers un dispositif de supervision, de données collectées par au moins un capteur comportant:
- Un module d'acquisition (506) d'une donnée mesurée par le au moins un capteur,
- Un calculateur (507) adapté pour calculer un indicateur d'écart entre la valeur de la nouvelle donnée et une valeur prédite pour cette donnée par un modèle de prédiction représentatif de données précédemment acquises,
- Un comparateur (505) adapté pour comparer l'indicateur d'écart à un seuil, et
- Un module de communication (504) adapté pour transmettre la nouvelle donnée vers le dispositif de supervision lorsque l'indicateur d'écart est supérieur au seuil, le dispositif étant **caractérisé en ce qu'**il comporte en outre
- Un module de réception d'un modèle de prédiction mis à jour à la base de la nouvelle donnée, un calcul ultérieur d'un indicateur d'écart se basant sur le modèle de prédiction mis à jour.

7. Dispositif de transmission selon la revendication 6 **caractérisé en ce que** le module de communication est en outre adapté pour recevoir, un modèle de prédiction représentatif des données transmises en provenance du dispositif de supervision.

8. Dispositif de supervision comportant les modules suivants :
- Un module de communication adapté pour recevoir des données en provenance d'au moins un capteur d'un système de supervision,
- Un module de lecture d'une valeur prédite par un modèle de prédiction représentatif de données de capteur précédemment reçues,
- Un module de supervision adapté pour remplacer une valeur de capteur non reçue par la valeur prédite tant qu'une nouvelle donnée dont un indicateur d'écart entre sa valeur et la valeur prédite pour cette donnée est supérieure à un seuil, n'est pas reçue par le module de communication
- Un module de réception adapté pour recevoir une nouvelle donnée issue du au moins un capteur,
- Un module de mise à jour du modèle prédictif,
- Un module de transmission du modèle mis à jour vers le au moins un capteur.

9. Dispositif de supervision selon la revendication 8 **caractérisé en ce qu'**il comporte en outre un calculateur adapté pour calculer un modèle prédictif représentatif de données reçues sur une période prédéterminée, et **en ce que** le module de communication est en outre adapté pour transmettre le modèle prédictif à au moins un dispositif de transmission.

10. Terminal **caractérisé en ce qu'**il comporte un dispositif de transmission selon l'une quelconque des revendications 6 ou 7.

11. Serveur **caractérisé en ce qu'**il comporte un dispositif de supervision selon l'une quelconque des revendications 8 ou 9.

12. Programme d'ordinateur comportant les instructions pour l'exécution des étapes du procédé de transmission selon l'une quelconque des revendications 1 à 2 et/ou les instructions pour l'exécution des étapes du procédé de supervision selon l'une quelconque des revendications 3 à 5, lorsque le programme est exécuté par un processeur.

13. Support d'informations lisible par un processeur sur lequel est enregistré un programme d'ordinateur comprenant des instructions pour l'exécution des étapes du procédé de transmission selon l'une quelconque des revendications 1 à 2 et/ou les instructions pour l'exécution des étapes du procédé de supervision selon l'une quelconque des revendications 3 à 5.

## Patentansprüche

1. Verfahren zur Übertragung von von mindestens einem Sensor gesammelten Daten an eine Überwachungsvorrichtung, das bei der Erfassung eines neuen Datenwerts durch den mindestens einen Sensor die folgenden Schritte aufweist:
- Berechnung (203) eines Abweichungsindikators zwischen dem Wert des neuen Datenwerts und einem für diesen Datenwert von einem für vorher erfasste Daten repräsentativen Vorhersagemodell vorhergesagten Wert, und
- Übertragung (204) des neuen Datenwerts an eine Überwachungsvorrichtung, wenn der Abweichungsindikator höher ist als eine Schwelle,
wobei das Verfahren **gekennzeichnet ist durch** den zusätzlichen Schritt des
- Empfangs eines auf der Basis des neuen Datenwerts aktualisierten Vorhersagemodells, wobei eine spätere Berechnung eines Abweichungsindikators auf dem aktualisierten Vorhersagemodell basiert.

2. Verfahren nach Anspruch 1, **dadurch gekennzeichnet, dass** es vorab die folgenden Schritte aufweist:
- Übertragung (201) von von dem mindestens einen Sensor über eine vorbestimmte Zeitperiode erfassten Daten an die Überwachungsvorrichtung, und
- Empfang (202) von der Überwachungsvorrichtung eines für die übertragenen Daten repräsentativen Vorhersagemodells.

3. Verfahren zur Überwachung durch einen Überwachungsserver ausgehend von von mindestens einem Sensor stammenden Daten, **dadurch gekennzeichnet, dass** es, um einen nicht empfangenen Sensorwert zu ersetzen, einen von einem für vorher empfangene Daten repräsentativen Vorhersagemodell vorhergesagten Wert berücksichtigt, so lange ein neuer Datenwert, von dem ein Abweichungsindikator zwischen seinem Wert und dem für diesen Datenwert vorhergesagten Wert höher ist als eine Schwelle, nicht empfangen wird, und dass es außerdem bei Empfang eines von dem mindestens einen Sensor stammenden neuen Datenwerts einen Schritt der Aktualisierung des Vorhersagemodells und einen Schritt der Übertragung des aktualisierten Modells an den mindestens einen Sensor aufweist.

4. Überwachungsverfahren nach Anspruch 3, **dadurch gekennzeichnet, dass** es die folgenden Schritte aufweist:
- Empfang (300) von Daten von dem mindestens einen Sensor über eine vorbestimmte Zeitperiode,
- Berechnung (301) eines für die in der Periode empfangenen Daten repräsentativen Vorhersagemodells, und
- Übertragung (302) des Vorhersagemodells an den mindestens einen Sensor.

5. Verfahren nach Anspruch 3, **dadurch gekennzeichnet, dass** der Schritt der Aktualisierung des Modells durchgeführt wird, wenn die Empfangsfrequenz neuer Daten höher ist als eine Schwelle.

6. Übertragungsvorrichtung von von mindestens einem Sensor gesammelten Daten an eine Überwachungsvorrichtung, die aufweist:
- ein Erfassungsmodul (506) eines Datenwerts, der von dem mindestens einen Sensor gemessen wird,
- einen Rechner (507), der geeignet ist, einen Abweichungsindikator zwischen dem Wert des neuen Datenwerts und einem für diesen Datenwert von einem für vorher erfasste Daten repräsentativen Vorhersagemodell vorhergesagten Wert zu berechnen,
- einen Komparator (505), der geeignet ist, den Abweichungsindikator mit einer Schwelle zu vergleichen, und
- ein Kommunikationsmodul (504), das geeignet ist, den neuen Datenwert an die Überwachungsvorrichtung zu übertragen, wenn der Abweichungsindikator höher ist als die Schwelle,
wobei die Vorrichtung **dadurch gekennzeichnet ist, dass** sie außerdem aufweist
- ein Empfangsmodul eines auf der Basis des neuen Datenwerts aktualisierten Vorhersagemodells, wobei eine spätere Berechnung eines Abweichungsindikators auf dem aktualisierten Vorhersagemodell basiert.

7. Übertragungsvorrichtung nach Anspruch 6, **dadurch gekennzeichnet, dass** das Kommunikationsmodul außerdem geeignet ist, ein für die von der Überwachungsvorrichtung kommenden übertragenen Daten repräsentatives Vorhersagemodell zu empfangen.

8. Überwachungsvorrichtung, die die folgenden Module aufweist:
- ein Kommunikationsmodul, das geeignet ist, Daten von mindestens einem Sensor eines Überwachungssystems zu empfangen,
- ein Lesemodul eines von einem für vorher empfangene Sensordaten repräsentativen Vorhersagemodell vorhergesagten Werts,
- ein Überwachungsmodul, das geeignet ist, einen nicht empfangenen Sensorwert durch den vorhergesagten Wert zu ersetzen, so lange ein neuer Datenwert, von dem ein Abweichungsindikator zwischen seinem Wert und dem für diesen Datenwert vorhergesagten Wert höher ist als eine Schwelle, nicht vom Kommunikationsmodul empfangen wird,
- ein Empfangsmodul, das geeignet ist, einen von dem mindestens einen Sensor stammenden neuen Datenwert zu empfangen,
- ein Aktualisierungsmodul des Vorhersagemodells,
- ein Übertragungsmodul des aktualisierten Modells an den mindestens einen Sensor.

9. Überwachungsvorrichtung nach Anspruch 8, **dadurch gekennzeichnet, dass** sie außerdem einen Rechner aufweist, der geeignet ist, ein für in einer vorbestimmten Periode empfangene Daten repräsentatives Vorhersagemodell zu berechnen, und dass das Kommunikationsmodul außerdem geeignet ist, das Vorhersagemodell an mindestens eine Übertragungsvorrichtung zu übertragen.

10. Endgerät, **dadurch gekennzeichnet, dass** es eine Übertragungsvorrichtung nach einem der Ansprüche 6 oder 7 aufweist.

11. Server, **dadurch gekennzeichnet, dass** er eine Überwachungsvorrichtung nach einem der Ansprüche 8 oder 9 aufweist.

12. Computerprogramm, das die Anweisungen zur Ausführung der Schritte des Übertragungsverfahrens nach einem der Ansprüche 1 bis 2 und/oder die Anweisungen zur Ausführung der Schritte des Überwachungsverfahren nach einem der Ansprüche 3 bis 5 aufweist, wenn das Programm von einem Prozessor ausgeführt wird.

13. Informationsträger, der von einem Prozessor lesbar ist, auf dem ein Computerprogramm aufgezeichnet ist, das Anweisungen zur Ausführung der Schritte des Übertragungsverfahrens nach einem der Ansprüche 1 bis 2 und/oder die Anweisungen zur Ausführung der Schritte des Überwachungsverfahrens nach einem der Ansprüche 3 bis 5 enthält.

## Claims

1. Method for transmitting, to a supervision device, data collected by at least one sensor comprising the following steps on acquisition of a new datum by the at least one sensor:
- calculation (203) of an indicator of deviation between the value of the new datum and a value predicted for this datum by a prediction model representative of data previously acquired, and
- transmission (204) of the new datum to a supervision device when the deviation indicator is above a threshold, the method being **characterized by** the additional step of
- reception of a prediction model updated on the basis of the new datum, a subsequent calculation of a deviation indicator being based on the updated prediction model.

2. Method according to Claim 1, **characterized in that** it comprises the following preliminary steps:
- transmission (201) to the supervision device of data acquired by the at least one sensor over a predetermined time period, and
- reception (202), from the supervision device, of a prediction model representative of the transmitted data.

3. Method for supervision by a supervision server based on data from at least one sensor, **characterized in that** it takes into account, to replace a sensor value that is not received, a value predicted by a prediction model representative of data previously received as long as a new datum, of which an indicator of deviation between its value and the value predicted for this datum is above a threshold, is not received and **in that** it comprises, in addition, on reception of a new datum from the at least one sensor, a step of updating of the predictive model and a step of transmission of the updated model to the at least one sensor.

4. Supervision method according to Claim 3, **characterized in that** it comprises the following steps:
- reception (300) of data from the at least one sensor over a predetermined time period,
- calculation (301) of a predictive model representative of the data received over the period, and
- transmission (302) of the predictive model to the at least one sensor.

5. Method according to Claim 3, **characterized in that** the step of updating of the model is performed when the frequency of reception of new data is above a threshold.

6. Device for transmitting, to a supervision device, data collected by at least one sensor comprising:
- an acquisition module (506) for acquiring a datum measured by the at least one sensor,
- a computer (507) suitable for calculating an indicator of deviation between the value of the new datum and a value predicted for this datum by a prediction model representative of data previously acquired,
- a comparator (505) suitable for comparing the deviation indicator to a threshold, and
- a communication module (504) suitable for transmitting the new datum to the supervision device when the deviation indicator is above the threshold, the device being **characterized in that** it further comprises
- a module for reception of a prediction model updated on the basis of the new datum, a subsequent calculation of a deviation indicator being based on the updated prediction model.

7. Transmission device according to Claim 6, **characterized in that** the communication module is also suitable for receiving a prediction model representative of the data transmitted from the supervision device.

8. Supervision device comprising the following modules:
- a communication module suitable for receiving data from at least one sensor of a supervision system,
- a module for reading a value predicted by a prediction model representative of sensor data previously received,
- a supervision module suitable for replacing a sensor value that is not received with the predicted value as long as a new datum for which an indicator of deviation between its value and the value predicted for this datum is above a threshold is not received by the communication module,
- a reception module suitable for receiving a new datum from the at least one sensor,
- a module for updating the predictive model,
- a module for transmitting the updated model to the at least one sensor.

9. Supervision device according to Claim 8, **characterized in that** it further comprises a computer suitable for calculating a predictive model representative of data received over a predetermined period, and **in that** the communication module is also suitable for transmitting the predictive model to at least one transmission device.

10. Terminal, **characterized in that** it comprises a transmission device according to either one of Claims 6 and 7.

11. Server, **characterized in that** it comprises a supervision device according to either one of Claims 8 and 9.

12. Computer program comprising instructions for the execution of the steps of the transmission method according to either one of Claims 1 and 2 and/or the instructions for the execution of the steps of the supervision method according to any one of Claims 3 to 5, when the program is run by a processor.

13. Processor-readable information medium on which is stored a computer program comprising instructions for the execution of the steps of the transmission method according to either one of Claims 1 and 2 and/or the instructions for the execution of the steps of the supervision method according to any one of Claims 3 to 5.
